(19) Europäisches Patentamt
European Patent Office
Office européen des brevets

(11) Veröffentlichungsnummer : **0 051 247**
**B1**

# (12) EUROPÄISCHE PATENTSCHRIFT

(45) Veröffentlichungstag der Patentschrift : **09.01.85**

(51) Int. Cl.⁴ : **C 07 D307/935**, A 61 K 31/34

(21) Anmeldenummer : **81108959.8**

(22) Anmeldetag : **27.10.81**

(54) **4-Thia- und 4-Sulfinyl-PGI1-Derivate, Verfahren zu deren Herstellung sowie diese Verbindungen enthaltende pharmazeutische Präparate.**

(30) Priorität : **28.10.80 HU 259680**

(43) Veröffentlichungstag der Anmeldung : **12.05.82 Patentblatt 82/19**

(45) Bekanntmachung des Hinweises auf die Patenterteilung : **09.01.85 Patentblatt 85/02**

(84) Benannte Vertragsstaaten : **AT BE CH DE FR GB IT LI NL SE**

(56) Entgegenhaltungen : **Keine**

(73) Patentinhaber : **CHINOIN Gyogyszer és Vegyészeti Termékek Gyára RT.**
**To utca 1-5**
**H-1045 Budapest IV (HU)**

(72) Erfinder : **Tömösközi, István, Dr.**
**Pozsonyi u. 6/a**
**H-1045 Budapest (HU)**
Erfinder : **Gyóri, Péter, Dr. Dipl. Ing. Chem.**
**Gyakorló u. 32**
**H-1106 Budapest (HU)**
Erfinder : **Kovács, Gábor, Dr. Dipl. Ing. Chem.**
**Róna park 4**
**H-1142 Budapest (HU)**
Erfinder : **Virág, Sándor, Dr.**
**Sallai I. u. 29/a**
**H-1136 Budapest (HU)**
Erfinder : **Körmöczy, Péter, Dr.**
**Uri u. 33**
**H-1014 Budapest (HU)**
Erfinder : **Stadler, István, Dr.**
**Mester u. 15**
**H-1095 Budapest (HU)**

(74) Vertreter : **Lotterhos, Hans Walter, Dr.-Ing.**
**Lichtensteinstrasse 3**
**D-6000 Frankfurt am Main 1 (DE)**

**EP 0 051 247 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue, biologisch aktive und therapeutisch verwendbare 4-Thia- und 4-Sulfinyl-$PGI_1$-Derivate der allgemeinen Formel

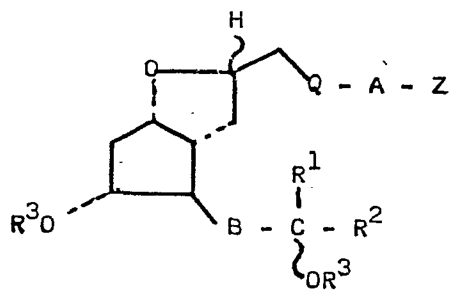

(I)

in der

Q —S— oder —SO—,

A geradkettiges oder verzweigtes $C_1$-$C_6$-Alkylen,

B Äthylen, Vinylen oder Äthinylen,

$R^1$ Wasserstoff oder $C_1$-$C_4$-Alkyl,

$R^2$ geradkettiges oder verzweigtes $C_1$-$C_8$-Alkyl oder gegebenenfalls durch Chlor oder Methoxy monosubstituiertes Aryloxymethyl,

$R^3$ Wasserstoff oder Acetyl und

Z —COOH, —CN, —$CH_2OH$ oder —COOW bedeuten, worin W ein Äquivalent eines pharmakologisch verträglichen Kations oder ein $C_1$-$C_4$-Alkyl ist.

In der allgemeinen Formel I kann das Wasserstoffatom des 3-Kohlenstoffatoms der 2-Oxa-bicyclo[3.3.0]octan-Struktur sterische α- oder β- (Exo oder Endo) Konfiguration haben. Die Erfindung umfaßt beide Epimer-Reihen. Außerdem kann die Hydroxylgruppe der Seitenkette am 6-Kohlenstoffatom der 2-Oxa-bicyclo[3.3.0]octan-Struktur S- oder R-Konfiguration aufweisen.

Die Verbindungen der Erfindung können in Form von Racematen oder optisch aktiven Enantiomeren vorliegen.

Ein wichtiger Vertreter der 1976 entdecken endogenen Prostaglandine ist das Prostacyclin ($PGI_2$), das eine bedeutende biologische Wirksamkeit aufweist (Prostacyclin, ed. : J.R. Vane und S. Bergström, Raven Press, N.Y., 1979).

Vom therapeutischen Standpunkt ist die die Thrombocytenaggregation hemmende, die peripheren Gefäße erweiternde und die cytoprotektive Wirkung des Prostacyclins sehr bedeutend. Die therapeutische Nutzung dieser biologischen Wirkungen wird jedoch durch die außerordentlich große Instabilität des Prostacyclins stark beschränkt. Seine Halbzeit in wäßriger Lösung bei neutralem pH beträgt nämlich nur 3,5 Minuten. Infolgedessen sind vom therapeutischen Standpukt dem Prostacyclin alle die Verbindungen vorzuziehen, die bei etwa gleicher biologischer Aktivität eine größere Stabilität als das Prostacyclin aufweisen.

Es wurde überraschenderweise gefunden, daß die Verbindungen der allgemeinen Formel I diesen Kriterien entsprechen. Sie hemmen die Thrombocytenaggregation, sie führen eine Erweiterung der vorzugsweise peripheren Gefäße und der Bronchien herbei, und sie üben eine Schutzwirkung auf die Magenschleimhaut aus. Sie zeigen damit praktisch die gleiche biologische Aktivität wie das Prostacyclin, zeichnen sich aber gleichzeitig durch eine viel größere Stabilität aus. Thermisch und hydrolytisch sind die Verbindungen der Erfindung in einem extrem großen pH-Bereich stabil. Außerdem zeigen die einzelnen Vertreter der Verbindungen der allgemeinen Formel I — je nach ihrer Struktur — auf den genannten Wirkungsgebieten eine höhere Selektivität als das Prostacyclin.

Die mit den Verbindungen der Erfindung erzielte Hemmwirkung auf die mit ADP (Adenosindiphosphat) bzw. Kollagen erzeugte Thrombocytenaggregation wurde nach Born untersucht. Die Wirkstoffkonzentration, die in 50 % die Aggregation des an Thrombocyten angereicherten Humanplasmas (PRP) hemmt, ist in der nachstehenden Tabelle angegeben.

| Aggregation erzeugende Mittel | 4-Thia-$PGI_1$-Natriumsalz | 4-Thia-$PGI_1$-Methylester |
|---|---|---|
| ADP | 200 ng/ml | 2,5 μg/ml |
| Kollagen | 2 μg/ml | 25 μg/ml |

Die haemodynamische Wirkung der Wirkstoffe wurde an Katzen untersucht. Den Ergebnissen ist zu entnehmen, daß das 4-Thia-$PGI_1$ erst in einer Dosis von 100 μg/kg den Kreislauf in dem gleichen Maße beeinflußt, wie $PGI_2$ in einer Dosis von 1 μg/kg. Dies zeigt, daß die unerwünschten Nebenwirkungen bei den Verbindungen der Erfindung wesentlich geringer sind.

Das 4-Thia-$PGI_1$ erzeugt eine Erschlaffung der Trachea des Meerschweinchens. Die Relaxation kann mit Inderal (1-Isopropylamino-3-[1-naphthyloxy]-propan-2-ol) nicht gehemmt werden. Das Maß der Relaxation betrug :

bei einer Dosis von 1 μg/ml 4-Thia-$PGI_1$ = 50 %,

bei einer Dosis von 10 μg/ml 4-Thia-$PGI_1$ = 83 %.

Die Erfindung betrifft auch pharmazeutische Präparate, die als Wirkstoff mindestens eine der Verbindungen der allgemeinen Formel I enthalten. Zur Herstellung der pharmazeutischen Präparate können die üblichen Hilfsstoffe, wie z. B. Füllmittel, Zusätze, die den Geschmack und den Geruch verbessern, die die Formulierung erleichtern, die den pH und den osmotischen Druck regulieren, Stabilisierungsmittel und/oder Resorptionsbeschleuniger, dem Wirkstoff oder den Wirkstoffen zugegeben werden. Die Präparate können, ebenso wie die Hilfsstoffe, fest, halb-flüssig oder flüssig sein. Als feste Präparate sind z. B. Tabletten, Kapseln, Pillen, Dragees, Pulver, als flüssige Präparate, Infusionen, Inhalations- und Injektionslösungen, Umschlaglösungen, Löffelarzneien, Tropfen usw. und als halbflüssige Präparate Creme, Salben, Suppositorien Balsam usw. zu nennen. Die Lösungen, Emulsionen können unter Verwendung bekannter Trägergase in Sprayform zubereitet werden.

Die erforderliche Dosierung der Wirkstoffe hängt von der Schwere der Erkrankung, der Geschwindigkeit der Resorption des Arzneimittels, der Empfindlichkeit des zu behandelnden Objektes oder Organs oder dessen Reaktionsfähigkeit ab. Sie variiert gewöhnlich zwischen etwa 1 μg/kg und 100 mg/kg.

Die Erfindung betrifft ferner die Herstellung der 4-Thia- und 4-Sulfinyl-$PGI_1$-Verbindungen der Formel I, wobei man vorzugsweise von einer Verbindung der allgemeinen Formel

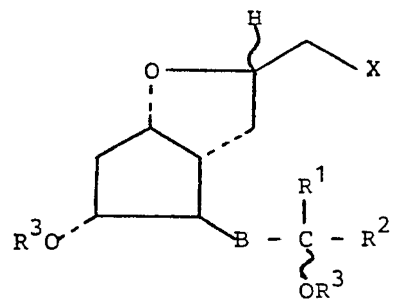

(II)

in der B, $R^1$, $R^2$ und $R^3$ die oben angegebene Bedeutung haben und X Brom oder Jod bedeutet, ausgeht. (Die Verbindungen der allgemeinen Formel II sind bekannt (Tetrahedron Lett., *1978,* 581)).

Hierbei wird gemäß Erfindung so verfahren, daß man die Verbindung der allgemeinen Formel II entweder

a) mit Thioharnstoff, Alkalimetallsulfid, Alkalimetallhydrogensulfid, Schwefelwasserstoff oder einem Salz der Mercaptoessigsäure umsetzt und mit Säure behandelt, wobei das Halogenatom der Halogenverbindung der allgemeinen Formel II durch —SH ersetzt wird, und das erhaltene Thiol der allgemeinen Formel

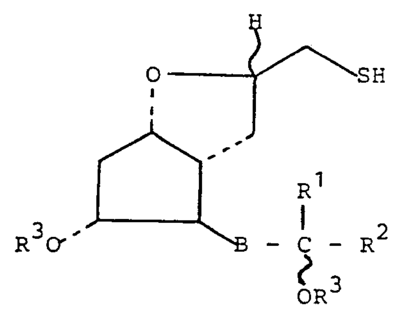

(III)

in der $R^1$, $R^2$, $R^3$ und B die oben angegebene Bedeutung haben, nach Zugabe von Basen mit einem Äthylenderivat der allgemeinen Formel

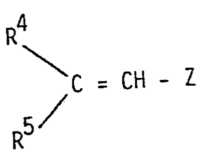

(V)

in der Z die oben angegebene Bedeutung hat und $R^4$ und $R^5$ unabhängig voneinander Wasserstoff oder $C_1$-$C_3$-Alkyl bedeuten oder mit einem Alkylhalogenid der allgemeinen Formel

X—A—Z (VI)

in der X, A und Z die oben angegebene Bedeutung haben, umsetzt, oder

b) zur Herstellung von Verbindungen der allgemeinen Formel I, in der Z —COOH ist, mit einem Salz einer Mercaptosäure der allgemeinen Formel

HS—A—COOH (VII)

in der A die oben angegebene Bedeutung hat, umsetzt, wobei die nach Verfahrensvariante a) oder b) erhaltene 4-Thia-Verbindung der allgemeinen Formel I (Q = —S—) gewünschtenfalls durch Oxydation in die entsprechende 4-Sulfinyl-Verbindung der allgemeinen Formel I (Q = —SO—) und/oder durch Veresterung, Amidierung, Salzbildung, Hydrolyse und/oder Reduktion in eine andere Verbindung der allgemeinen Formel I übergeführt werden kann.

Gemäß Verfahrensvariante a) werden die Verbindungen der allgemeinen Formel II — vorzugsweise in $C_1$-$C_4$-Alkanolen oder deren wäßrigen Gemischen — bei 25 bis 120 °C mit Thioharnstoff, Alkalimetallsulfid, Alkalimetallhydrogensulfid oder Schwefelwasserstoff umgesetzt und das entstandene Produkt durch basische Hydrolyse und darauf folgendes Ansäuern oder saure Behandlung in ein Thiol der allgemeinen Formel III übergeführt.

Nach einer bevorzugten Verfahrensweise werden die Halogenverbindungen der allgemeinen Formel II mit einem Salz der Mercaptoessigsäure, vorzugsweise mit Natrium-mercaptoacetat, umgesetzt, wobei Verbindungen der allgemeinen Formel

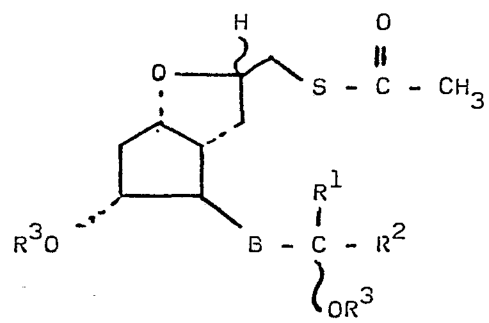

(IV)

in der B, $R^1$, $R^2$ und $R^3$ die oben angegebene Bedeutung haben, entstehen, die nach alkalischer Hydrolyse die Thiole der allgemeinen Formel III liefern.

Die Thiole der allgemeinen Formel III werden entweder mit Äthylenverbindungen der allgemeinen Formel V in Gegenwart einer katalytischen Menge von Alkalimetallhydroxyden oder Alkylimetallalkoholaten, vorzugsweise Natriumäthylat, oder tertiären Aminen, vorzugsweise Triäthylamin oder Pyridin, oder mit Alkylhalogeniden der allgemeinen Formel VI in Gegenwart einer äquivalenten oder etwas überschüssigen Menge an Base in $C_1$-$C_4$-Alkanol oder vorzugsweise in Aceton umgesetzt.

Gemäß Verfahrensvariante b) wird die Umsetzung in $C_1$-$C_4$-Alkanolen, z. B. Äthanol, oder in einem Gemisch von Alkanolen und Wasser vorzugsweise bei 25 bis 70 °C durchgeführt.

Die Salze der Mercaptocarbonsäuren sind ambidente nucleophile Reagenzien. Da aber die Reaktion des « weichen » Thiolat-Ions an dem Kohlenstoffatom, das ebenfalls ein « weiches » elektrophiles Halogenatom trägt, sehr viel stärker « begünstigt » als die des « harten » nucleophilen Carboxylations ist, entsteht nur die Verbindung der allgemeinen Formel I (Diese Reaktion ist in J. Chem. Educ. *1968,* 45, S. 581 und 683 ausführlich beschrieben).

Die erhaltenen 4-Thia-Verbindungen der allgemeinen Formel I (Q = —S—) werden in bekannten Lösungsmitteln, vorzugsweise in chlorierten Kohlenwasserstoffen, z. B. Dichlormethan, durch Oxydation mit organischen Persäuren in die entsprechenden 4-Sulfinyl-Verbindungen der allgemeinen Formel I (Q = —SO—) übergeführt. Als Persäuren können vorzugsweise m-Chlor-perbenzoesäure oder Peressigsäure verwendet werden.

Die als zusätzliche Reaktionen der Verbindungen der allgemeinen Formel I angegebenen Reaktionen, wie z. B. die Veresterung, Salzbildung, Amidierung, Hydrolyse oder Reduktion können in an sich bekannter Weise durchgeführt werden. So können z. B. in den erhaltenen Verbindungen die Estergruppen durch alkalische Hydrolyse in die freien Carboxylgruppen, bzw. deren Salz oder in die freien Hydroxylgruppen übergeführt werden. Die freien Carboxylgruppen können in an sich bekannter Weise verestert bzw. amidiert werden. Nach Dehydrierung der Amide kann man Nitrile und aus den Nitrilen bzw. Amiden kann man durch Hydrolyse freie Säuren erhalten.

Weitere Einzelheiten der Erfindung sind den folgenden Beispielen zu entnehmen.

## Beispiel 1

3β-(Acetylmercapto-methyl)-6β-(3α-acetoxy -oct-1-trans-enyl)-7α-acetoxy-cis-2-oxa-bicyclo[3.3.0.]octan (Verbindung der Formel IV : B = trans-Vinylen, $R^1$ = Wasserstoff, $R^2$ = n-Pentyl, $R^3$ = Acetyl.)

In 10 ml wasserfreiem Aceton löst man 0,956 g (0,002 Mol) 3β-Jodmethyl-6β-(3α-acetoxy-oct-1-trans-

enyl)-7α-acetoxy-cis-2-oxa-bicyclo[3.3.0]octan (Verbindung der Formel II : X = Jod ; B, $R^1$, $R^2$, $R^3$ wie oben angegeben), gibt unter Rühren 0,21 g (0,204 Mol) Natriummercaptoacetat zu und erhitzt das Reaktionsgemisch unter Rühren auf einem Bad von 60 °C. Die Reaktion wird mittels Dünnschichtchromatographie beobachtet. Der $R_f$-Wert der Ausgangsverbindung beträgt 0,53 und der $R_f$-Wert des Endproduktes beträgt 0,47 an Kieselgel (Elution mit einem 1 : 1 Gemisch von Hexan und Äthylacetat). Nach Verbrauch der Ausgangsverbindung wird das Reaktionsgemisch auf 40 ml Wasser von 0 °C gegossen, 3-mal mit je 50 ml Äther extrahiert, die Ätherlösung 2-mal mit je 20 ml kaltem Wasser, mit 20 ml gesättigter Natriumchloridlösung gewaschen, über Magnesiumsulfat getrocknet und eingeengt. Die Titelverbindung wird in Form eines zähen Öls in einer Menge von 0,8-0,85 g (94-99 %) erhalten. Das Endprodukt braucht für die weitere Verarbeitung nicht gereinigt zu werden, aber es kann — falls erforderlich — an Kieselgel chromatographiert und mit einem 1 : 2 Gemisch von Äthylacetat und Hexan eluiert werden.

$R_f$ : 0,26 (1 : 2 Gemisch von Äthylacetat und Hexan)

$H^1$-NMR($CDCl_3$) : 5,48 (m, 2H), 5,18 (m, 1H), 4,75 (q, 1H), 4,48 (m, 1H), 4,15 (m, 1H), 3,09 (dd, 2H), 2,34 (s, 3H), 2,03 (s, 3H), 2,00 (s, 3H), 0,87 (t, 3H) ppm.

## Beispiel 2

3β-Mercaptomethyl-6β-(3α-hydroxy-oct-1-trans-enyl)-7α-hydroxy-cis-2-oxa-bicyclo[3.3.0]octan (Verbindung der Formel III : B = trans-Vinylen, $R^1$ und $R^3$ = Wasserstoff, $R^2$ = n-Pentyl.)

Man löst 1,472 g 3β-Jodmethyl-6β-(3α-hydroxy-oct-1-trans-enyl)-7α-hydroxy-cis-2-oxa-bicyclo[3.3.0]octan (Verbindung der Formel II : X = Jod ; B, $R^1$, $R^2$, $R^3$ wie oben angegeben) in 5 ml wasserfreiem Äthanol, gibt 0,3 g Thioharnstoff zu der Lösung und erhitzt die Lösung unter Rühren und unter Rückfluß bis zum Verbrauch der Ausgangsverbindung. Die Reaktion ist nach 60 bis 80 Stunden beendet. Der $R_f$-Wert der Jodverbindung der Formel II beträgt 0,38 (Elution mit einem 1 : 1 Gemisch von Hexan und Aceton). Das gebildete Thiuronium-Salz bleibt praktisch am Startpunkt. Nach Beendigung der Reaktion wird das Äthanol durch Einengen auf einem Rotationsverdampfer entfernt, der Rückstand in 5 ml einer 1 M wäßrigen Natriumhydroxydlösung gelöst und 4 Stunden bei 80 °C gerührt.

Nach Abkühlen verdünnt man das Reaktionsgemisch mit 10 ml Wasser, säuert es mit 2 ml 1 M wäßriger Natriumhydrogensulfatlösung an und extrahiert es 3-mal mit je 30 ml Äther. Die Ätherlösung wird mit 10 ml gesättigter Natriumchloridlösung gewaschen und über Magnesiumsulfat getrocknet. Nach dem Abdestillieren des Lösungsmittels erhält man 1,063 g (94,7 %) der Titelverbindung als Öl. $R_f$ = 0,33, Elutionsmittel : 1 : 1 Gemisch von Hexan und Acetat.

Das erhaltene Produkt liefert nach Behandeln mit 2 ml Acetanhydrid in 5 ml Pyridin bei Raumtemperatur für 14 Stunden ein mit dem Produkt des Beispiels 1 identisches Triacetylderivat.

## Beispiel 3

4-Thia-β-$PGI_1$-methylester

(Verbindung der Formel I : Q = —S—, A = —$CH_2$—$CH_2$—, B = trans-Vinylen, $R^1$ und $R^3$ = Wasserstoff, $R^2$ = n-Pentyl, Z = —$COOCH_3$)

0,601 g (0,022 Mol) des in Beispiel 2 erhaltenen Produktes löst man in 20 ml wasserfreiem Äthanol und versetzt die Lösung zunächst mit 0,8 ml Acylsäuremethylester und dann unter Rühren mit ein paar Tropfen einer 5 %igen äthanolischen Natriumäthylatlösung. Die Reaktion ist bei Raumtemperatur in 1 bis 2 Stunden beendet. Der Verlauf der Reaktion wird an einer Kieselgel-Dünnschicht mit einem 1 : 1 Gemisch von Hexan und Aceton als Elutionsmittel beobachtet. $R_f$-Wert der Zielverbindung : 0,33 und der Ausgangsverbindung : 0,27. Nach Beendigung der Reaktion wird das Äthanol aus dem Reaktionsgemisch durch Eindampfen auf einem Rotationsverdampfer entfernt und der Rückstand an einer 100 g Kieselgelsäule mit einem 1 : 1 Gemisch von Hexan und Aceton als Elutionsmittel chromatographiert.

Man erhält 0,678 g (88 %) 4-Thia-β-$PGI_1$-methylester.

$H^1$-NMR($CDCl_3$) : 5,5 (m, 2H), 4,43 (q, 1H), 4,16 (m, 1H), 4,06 (m, 1H), 3,66 (s, 3H), 3,61 (m, 1H), 2,84-2,85 (m, 7H), 2,5-1,15 (m, 15H), 0,84 (t, 3H) ppm.

## Beispiel 4

3(R,S)-Methyl-4-thia-β-$PGI_1$-methylester

(Verbindung der Formel 1 : A = —$CH(CH_3)$—$CH_2$—, Q, B, $R^1$, $R^2$, $R^3$, Z wie in Beispiel 3)

Es wird wie in Beispiel 3 verfahren aber statt Acrylsäuremethylester 0,8 ml Crotonsäuremethylester verwendet. Die Titelverbindung erhält man in einer Ausbeute von 66 % in Form eines chromatographisch nicht trennbaren Epimerengemisches. Die Komponenten unterscheiden sich in der absoluten sterischen Konfiguration des Methylsubstituenten am 3-Kohlenstoffatom. $R_f$ = 0,31, mit einem 1 : 1 Gemisch von Hexan und Aceton.

$H^1$-NMR($CDCl_3$) : 5,53 (m, 2H), 4,45 (q, 1H), 4,25-3,97 (m, 2H), 3,67 (s, 3H), 3,60 (m, 1H), 3,22 (m, 1H), 2,56-1,18 (m, 20H), 1,33 (2d, 3H, J = 7,5 Hz), 0,87 (t, 3H) ppm.

Beispiel 5

4-Thia-β-$PGI_1$-nitril

(Verbindung der Formel I : Z = —C ≡ N, Q, A, B, $R^1$, $R^2$, $R^3$ wie in Beispiel 3 angegeben)

Es wird wie in Beispiel 3 verfahren aber statt Acrylsäuremethylester 0,8 ml Acrylonitril verwendet. Man erhält 0,629 g (89 %) der Titelverbindung in Form eines farblosen Öls. $R_f$ = 0,21. Elutionsmittel : 1 : 1 Gemisch von Aceton und Hexan.

IR (Film) : 3 300-3 100 (assoziiertes OH), 2 190 (C ≡ N) $cm^{-1}$

Beispiel 6

4-Thia-β-$PGI_1$

(Verbindung der allgemeinen Formel I : Z = —COOH, Q, A, B, $R^1$, $R^2$, $R^3$ wie in Beispiel 3)

0,386 g (0,001 Mol) des nach Beispiel 3 hergestellten 4-Thia-β-$PGI_1$-methylesters löst man in 10 ml Methanol, gibt unter Rühren 2 ml 0,5 M Natriumhydroxydlösung zu und läßt das Reaktionsgemisch 24 Stunden bei Raumtemperatur stehen. Während dieser Zeit wird der Ester vollkommen verseift (auf dem Dünnschichtchromatogramm — Elutionsmittel = 1 : 1 Gemisch von Hexan und Aceton — verschwindet der dem Ester entsprechende Fleck $R_f$ = 0,27 und ein sich von dem Startpunkt kaum bewegender Fleck erscheint). Danach wird das Methanol im Vakuum auf einem Rotationsverdampfer entfernt, der erhaltene Rückstand mit 1 ml 1 M wäßriger Natriumhydrogensulfatlösung angesäuert und mit 30 ml eines 1 : 1 Gemisches von Äther und Äthylacetat extrahiert. Die so erhaltene organische Lösung wird 2-mal mit je 5 ml gesättigter Natriumchloridlösung gewaschen und über Magnesiumsulfat getrocknet. Nach Entfernung des Lösungsmittels erhält man als Rückstand 0,360 g (96,6 %) der Titelverbindung als kristalline Substanz, die aus einem Gemisch von Äther und Hexan oder Äthylacetat und Hexan umkristallisiert werden kann.

$R_f$ = 0,18, Elutionsmittel : 20 : 10 : 1 Gemisch von Benzol, Dioxan und Essigsäure.

Beispiel 7

4-Sulfinyl-β-$PGI_1$-methylester

(Verbindung der Formel I : Q = —SO—, A, B, $R^1$, $R^2$, $R^3$, Z wie in Beispiel 3)

Man löst 1,00 g (0,002 6 Mol) des nach Beispiel 3 hergestellten 4-Thia-β-$PGI_1$-methylesters in 20 ml wasserfreiem Dichlormethan, kühlt die Lösung auf 0 °C und gibt unter Rühren in kleinen Portionen 0,55 g (0,003 2 Mol) 3-Chlor-perbenzoesäure innerhalb von 15 Minuten zu. Nach weiterem Rühren von 30 Minuten wird die Lösung in einem Schütteltrichter mit 5 ml gesättigter Natriumhydrogencarbonatlösung, 5 ml 5 %iger Natriumhydrogensulfatlösung und 2 mal mit je 5 ml Wasser ausgeschüttelt bzw. gewaschen. Das Gemisch wird über Magnesiumsulfat getrocknet und das Lösungsmittel im Vakuum entfernt. Man erhält 0,845 g (81 %) Rohprodukt.

$R_f$ = 0,106, an Kieselgel ; Elutionsmittel : 1 : 2 Gemisch von Aceton und Hexan.

**Ansprüche** (für die Vertragsstaaten : BE, CH, DE, FR, GB, IT, LI, NL, SE)

1. 4-Thia- und 4-Sulfinyl-$PGI_1$-Derivate der allgemeinen Formel

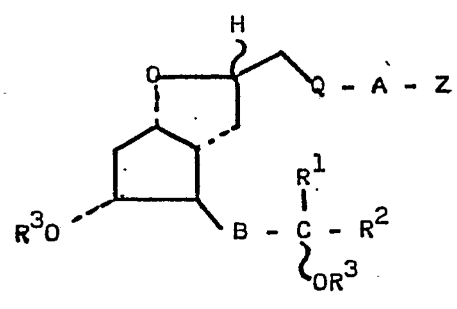

(I)

in der

Q —S— oder —SO—,

A geradkettiges oder verzweigtes $C_1$-$C_6$-Alkylen,

B Äthylen, Vinylen oder Äthinylen,

$R^1$ Wasserstoff oder $C_1$-$C_4$-Alkyl,

$R^2$ geradkettiges oder verzweigtes $C_1$-$C_8$-Alkyl oder gegebenenfalls durch Chlor oder Methoxy monosubstituiertes Aryloxymethyl,

$R^3$ Wasserstoff oder Acetyl und

Z —COOH, —CN, —$CH_2OH$ oder —COOW bedeuten, worin W ein Äquivalent eines pharmakologisch verträglichen Kations oder ein $C_1$-$C_4$-Alkyl ist.

2. 4-Thia-β-$PGI_1$-methylester.
3. 3-(R,S)-Methyl-4-thia-β-$PGI_1$-methylester.
4. 4-Thia-β-$PGI_1$.
5. 4-Thia-β-$PGI_1$-nitril.
6. 4-Sulfinyl-β-$PGI_1$-methylester.
7. Verfahren zur Herstellung von 4-Thia- und 4-Sulfinyl-$PGI_1$-Derivaten der allgemeinen Formel

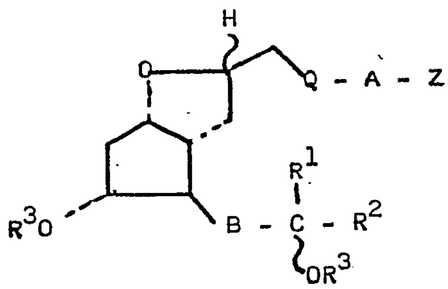

(I)

in der Q, A, B, $R^1$, $R^2$, $R^3$ und Z die oben angegebene Bedeutung haben, dadurch gekennzeichnet, daß man eine Halogenverbindung der allgemeinen Formel

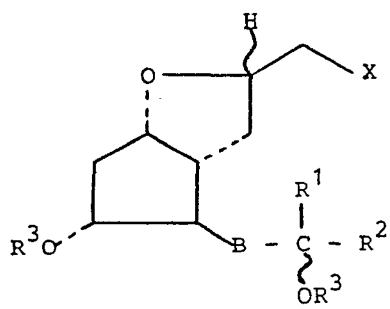

(II)

in der
$R^1$, $R^2$, $R^3$ und B die oben angegebene Bedeutung haben und
X Brom oder Jod ist,
entweder
a) mit Thioharnstoff, Alkalimetallsulfid, Alkalimetallhydrogensulfid, Schwefelwasserstoff oder einem Salz der Mercaptoessigsäure umsetzt, ansäuert und das erhaltene Thiol der allgemeinen Formel

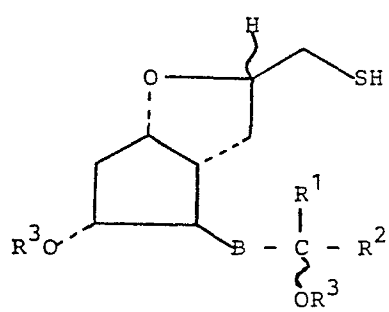

(III)

in der $R^1$, $R^2$, $R^3$ und B die oben angegebene Bedeutung haben nach Zugabe von Basen mit einem Äthylenderivat der allgemeinen Formel

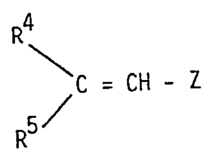

(V)

in der

Z die oben angegebene Bedeutung hat und

$R^4$ und $R^5$ unabhängig voneinander Wasserstoff oder $C_1$-$C_3$-Alkyl bedeuten, oder mit einem Alkylhalogenid der allgemeinen Formel

X—A—Z (VI)

in der A, Z und X die oben angegebene Bedeutung haben, umsetzt,

oder

b) zur Herstellung von Verbindungen der allgemeinen Formel I in der Z —COOH ist, mit einem Salz einer Mercaptocarbonsäure der allgemeinen Formel

HS—A—COOH (VII)

in der A die oben angegebene Bedeutung hat, umsetzt, wobei die erhaltene 4-Thia-β-$PGI_1$-Verbindung der allgemeinen Formel I gewünschtenfalls durch Oxydation in die entsprechende 4-Sulfinyl-$PGI_1$-Verbindung der allgemeinen Formel I und/oder durch Veresterung, Salzbildung, Amidierung, Hydrolyse und/oder Reduktion in eine andere Verbindung der allgemeinen Formel I übergeführt werden.

8. Verfahren nach Anspruch 7, dadurch gekennzeichnet, daß man die Oxydation der 4-Thia-β-$PGI_1$-Verbindung der allgemeinen Formel I zur entsprechenden 4-Sulfinyl-$PGI_1$-Verbindung mit 3-Chlorperbenzoesäure durchführt.

9. Pharmazeutische Präparate, die als Wirkstoff eine oder mehrere Verbindungen der Ansprüche 1 bis 6 enthalten.

**Ansprüche** (für den Vertragsstaat AT)

1. Verfahren zur Herstellung von 4-Thia- und 4-Sulfinyl-$PGI_1$-Derivaten der allgemeinen Formel

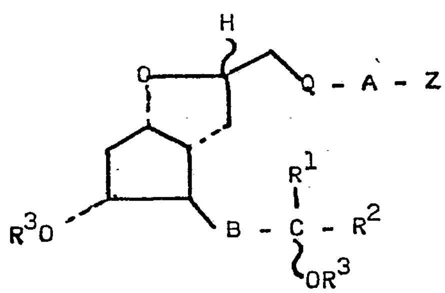

(I)

in der

Q —S— oder —SO—,

A geradkettiges oder verzweigtes $C_1$-$C_6$-Alkylen,

B Äthylen, Vinylen oder Äthinylen,

$R^1$ Wasserstoff oder $C_1$-$C_4$-Alkyl,

$R^2$ geradkettiges oder verzweigtes $C_1$-$C_8$-Alkyl oder gegebenenfalls durch Chlor oder Methoxy monosubstituiertes Aryloxymethyl,

$R^3$ Wasserstoff oder Acetyl und

Z —COOH, —CN, —$CH_2$OH oder —COOW bedeuten, worin W ein Äquivalent eines pharmakologisch verträglichen Kations oder ein $C_1$-$C_4$-Alkyl ist,

dadurch gekennzeichnet, daß man eine Halogenverbindung der allgemeinen Formel

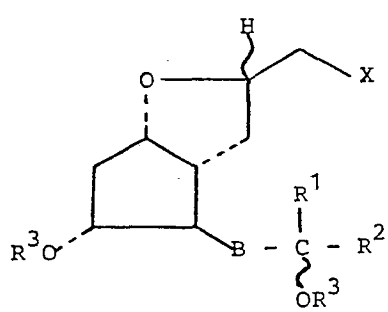

(II)

in der

$R^1$, $R^2$, $R^3$ und B die oben angegebene Bedeutung haben und

X Brom oder Jod ist,

entweder

a) mit Thioharnstoff, Alkalimetallsulfid, Alkalimetallhydrogensulfid, Schwefelwasserstoff oder einem Salz der Mercaptoessigsäure umsetzt, ansäuert und das erhaltene Thiol der allgemeinen Formel

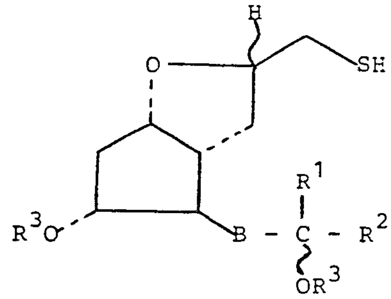

(III)

in der $R^1$, $R^2$, $R^3$ und B die oben angegebene Bedeutung haben nach Zugabe von Basen mit einem Äthylenderivat der allgemeinen Formel

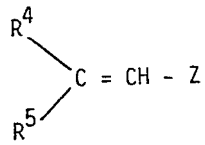

(V)

in der

Z die oben angegebene Bedeutung hat und

$R^4$ und $R^5$ unabhängig voneinander Wasserstoff oder $C_1$-$C_3$-Alkyl bedeuten, oder mit einem Alkylhalogenid der allgemeinen Formel

X—A—Z (VI)

in der A, Z und X die oben angegebene Bedeutung haben, umsetzt,

oder

b) zur Herstellung von Verbindungen der allgemeinen Formel I

in der

Z —COOH ist,

mit einem Salz einer Mercaptocarbonsäure der allgemeinen Formel

HS—A—COOH (VII)

in der A die oben angegebene Bedeutung hat,

umsetzt, wobei die erhaltene 4-Thia-β-$PGI_1$-Verbindung der allgemeinen Formel I gewünschtenfalls durch Oxydation in die entsprechende 4-Sulfinyl-$PGI_1$-Verbindung der allgemeien Formel I und/oder durch Veresterung, Salzbildung, Amidierung, Hydrolyse und/oder Reduktion in eine andere Verbindung der allgemeinen Formel I übergeführt werden.

2. Verfahren nach Anspruch 1, dadurch gekennzeichnet, daß man die Oxydation der 4-Thia-β-$PGI_1$-Verbindung der allgemeinen Formel I zur entsprechenden 4-Sulfinyl-$PGI_1$-Verbindung mit 3-Chlorperbenzoesäure durchführt.

3. Verwendung von einer oder mehreren gemäß Anspruch 1 oder 2 erhaltenen Verbindung(en) zur Herstellung von pharmazeutischen Präparaten.

**Claims** (for the Contracting States : BE, CH, DE, FR, GB, IT, LI, NL, SE)

1. 4-thia and 4-sulfinyl-$PGI_1$ derivates having the general formula

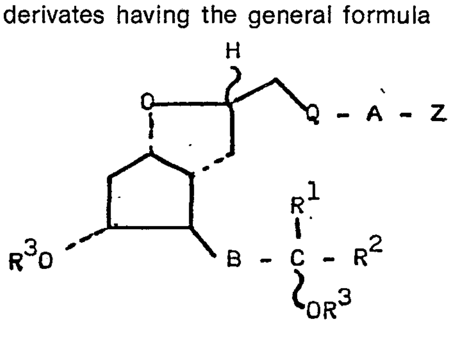

(I)

in which

Q = —S— or —SO—,

A = straight-chain or branched $C_1$-$C_6$ alkylene,

B = ethylene, vinylene or ethynylene

$R^1$ = hydrogen or $C_1$-$C_4$ alkyl,

$R^2$ = straight-chain or branched $C_1$-$C_8$ alkyl or aryloxymethyl, monosubstituted if required by chlorine or methoxy

$R^3$ = hydrogen or acetyl and

Z = —COOH, —CN, —$CH_2$OH or —COOW where W is an equivalent of a pharmacologically compatible cation or a $C_1$-$C_4$ alkyl.

2. 4-thia-β-$PGI_1$ methylester.
3. 3-(R,S)-methyl-4-thia-β-$PGI_1$-methylester.
4. 4-thia-β-$PGI_1$.
5. 4-thia-β-$PGI_1$-nitrile.
6. 4-sulfinyl-β-$PGI_1$-methylester.
7. A method of preparing 4-thia and 4-sulfinyl $PGI_1$ derivatives having the general formula :

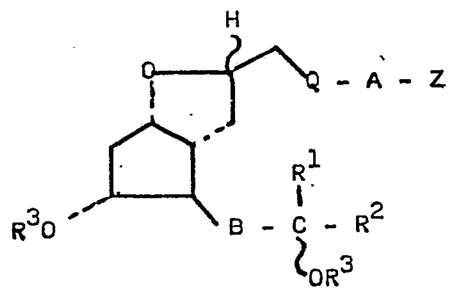

(I)

in which Q, A, B, $R^1$, $R^2$, $R^3$ and Z have the previously-given meanings, characterised in that a halogen compound having the general formula

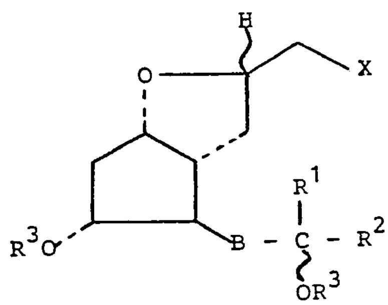

(II)

in which

$R^1$, $R^2$, $R^3$ and B have the previously-given meanings and

X = bromine or iodine is reacted either

a) with thiourea, alkali metal sulfide, alkali metal hydrogen sulfide, hydrogen sulfide or a salt of mercaptoacetic acid, and is acidified and the resulting thiol having the general formula

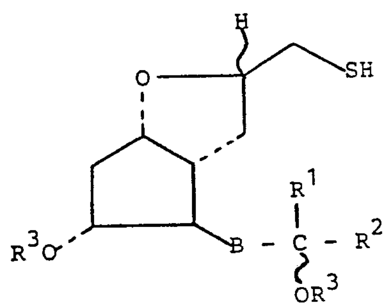

(III)

in which $R^1$, $R^2$, $R^3$ and B have the previously-given meanings, is mixed with bases and reacted with an ethylene derivative having the general formula

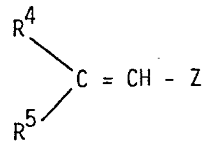

(V)

in which

Z has the previously-given meaning and

$R^4$ und $R^5$ independently denote hydrogen or $C_1$-$C_3$ alkyl, or with an alkyl halide having the general formula

$$X—A—Z \qquad (VI)$$

in which A, Z and X have the previously-given meanings,

or

b) in order to produce compounds having the general formula I in which Z = —COOH, the substance is reacted with a salt of a mercaptocarboxylic acid having the general formula

$$HS—A—COOH \qquad (VII)$$

in which A has the previously-given meaning, and if required the resulting 4-thia-β-$PGI_1$ compound having the general formula I is converted by oxidation into the corresponding 4-sulfinyl-$PGI_1$ compound having the general formula I and/or by esterification, salt formation, amidation, hydrolysis and/or reduction into another compound having the general formula I.

8. A method according to Claim 7 characterised in that 3-chloroperbenzoic acid is used for oxidising the 4-thia-β-$PGI_1$ compound having the general formula I to form the corresponding 4-sulfinyl-$PGI_1$ compound.

9. Pharmaceutical preparations in which the active principle is one or more compounds according to Claims 1 to 6.

**Claims** (for the Contracting State AT)

1. A method of preparing 4-thia and 4-sulfinyl-$PGI_1$ derivatives having the general formula

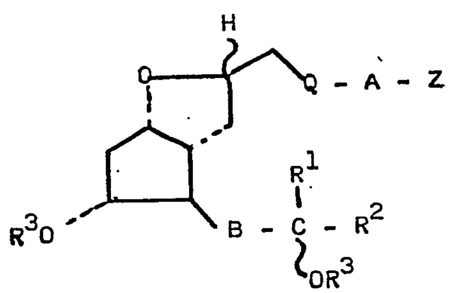

(I)

in which

Q = —S— or —SO—,

A = straight-chain or branched $C_1$-$C_6$ alkylene,

B = ethylene, vinylene or ethynylene

$R^1$ = hydrogen or $C_1$-$C_4$ alkyl,

$R^2$ = straight-chain or branched $C_1$-$C_8$ alkyl or aryloxymethyl, monosubstituted if required by chlorine or methoxy

$R^3$ = hydrogen or acetyl and

Z = —COOH, —CN, —$CH_2$OH or —COOW

where W is an equivalent of a pharmacologically compatible cation or a $C_1$-$C_4$ alkyl.

characterised in that a halogen compound having the general formula

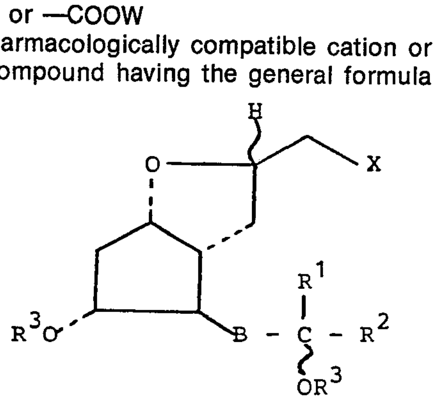

(II)

in which

$R^1$, $R^2$, $R^3$ and B have the previously-given meanings and

X = bromine or iodine is reacted either

a) with thiourea, alkali metal sulfide, alkali metal hydrogen sulfide, hydrogen sulfide or a salt of mercaptoacetic acid, and is acidified and the resulting thiol having the general formula

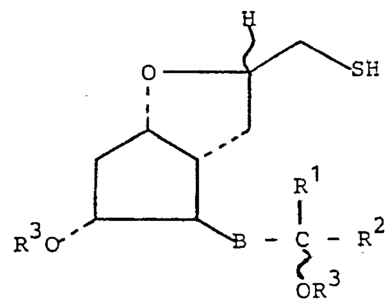

(III)

in which $R^1$, $R^2$, $R^3$ and B have the previously-given meanings, is mixed with bases and reacted with an ethylene derivative having the general formula

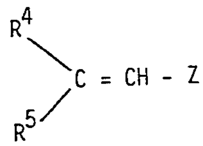

(V)

in which

Z has the previously-given meaning and

$R^4$ and $R^5$ independently denote hydrogen or $C_1$-$C_3$ alkyl or with an alkyl halide having the general formula

$$X—A—Z \qquad (VI)$$

in which A, Z and X have the previously-given meanings,

or

b) in order to produce compounds having the general formula I in which Z = —COOH, the substance is reacted with a salt of a mercaptocarboxylic acid having the general formula

$$HS—A—COOH \qquad (VII)$$

in which A has the previously-given meaning,

and if required the resulting 4-thia-β-$PGI_1$ compound having the general formula I is converted by oxidation into the corresponding 4-sulfinyl-$PGI_1$ compound having the general formula I and/or by esterification, salt formation, amidation, hydrolysis and/or reduction into another compound having the general formula I.

2. A method according to Claim 1 characterised in that 3-chloroperbenzoic acid is used for oxidising the 4-thia-β-$PGI_1$ compound having the general formula I to form the corresponding 4-sulfinyl-$PGI_1$ compound.

3. Use of one or more of the compounds obtained according to Claim 1 or 2 for producing pharmaceutical preparations.

**Revendications** (pour les Etats contractants : BE, CH, DE, FR, GB, IT, LI, NL, SE)

1. Dérivés 4-thia- et 4-sulfinyl-$PGI_1$ de formule générale I

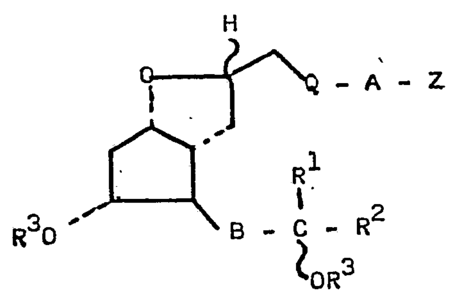

(I)

dans laquelle

Q représente —S— ou —SO—,

A représente un groupe alkylène en $C_1$ à $C_6$ linéaire ou ramifié,

B représente un groupe éthylène, vinylène ou éthynylène,

$R^1$ représente de l'hydrogène ou un radical alkyl en $C_1$ à $C_4$,

$R^2$ représente un radical alkyl en $C_1$ à $C_8$ linéaire ou ramifié, ou un radical aryloxyméthyl éventuellement monosubstitué par un atome de chlore ou un groupe méthoxy,

$R^3$ représente de l'hydrogène ou un radical acétyl, et

Z représente un radical —COOH, —CN, —$CH_2$OH ou —COOW où W représente l'équivalent d'un cation pharmaceutiquement acceptable ou un radical alkyl en $C_1$ à $C_4$.

2. L'ester méthylique de 4-thia-β-$PGI_1$.
3. L'ester méthylique de 3-(R,S)-méthyl-4-thia-β-$PGI_1$.
4. Le 4-thia-β-$PGI_1$.
5. Le nitrile de 4-thia-β-$PGI_1$.
6. L'ester méthylique de 4-sulfinyl-β-$PGI_1$.
7. Procédé pour la préparation de dérivés 4-thia- et 4-sulfinyl-$PGI_1$ de formule générale I

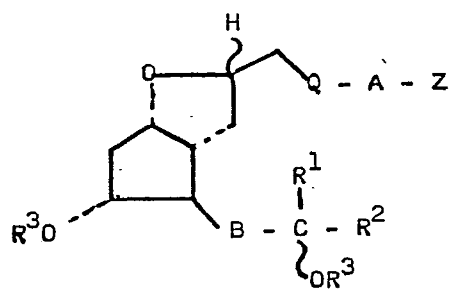

(I)

dans laquelle

Q, A, B, $R^1$, $R^2$, $R^3$ et Z ont les significations données précédemment, caractérisé en ce qu'un composé halogéné de formule générale II

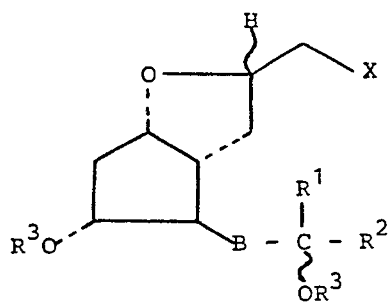

(II)

dans laquelle

$R^1$, $R^2$, $R^3$ et B ont les significations données précédemment, et

X représente un atome de brome ou d'iode,

a) est mis en réaction avec une thiourée, un sulfure de métal alcalin, un hydrogénosulfure de métal alcalin, de l'hydrogène sulfuré ou un sel d'acide mercaptoacétique, en ce que l'on acidifie et en ce que le thiol obtenu, répondant à la formule générale III

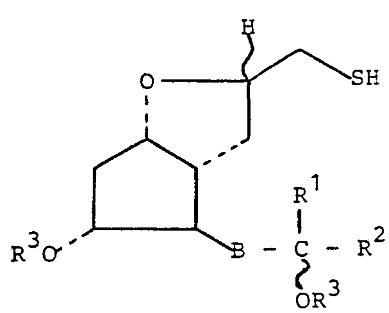

(III)

dans laquelle

$R^1$, $R^2$, $R^3$ et B ont les significations données précédemment, après addition de base, est mis en réaction avec un dérivé éthylénique de formule générale V

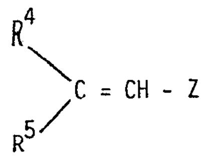

(V)

dans laquelle

Z a la signification donnée précédemment, et

$R^4$ et $R^5$ représentent indépendamment l'un de l'autre de l'hydrogène ou un groupe alkyle en $C_1$ à $C_3$, ou avec un halogénure d'alkyl de formule générale VI

X—A—Z (VI)

dans laquelle

A, Z et X ont les significations données précédemment, ou bien

b) pour obtenir un dérivé de formule générale I, dans laquelle Z représente le radical —COOH, est mis en réaction avec un sel d'un acide mercaptocarboxylique de formule générale VII

HS—A—COOH (VII)

dans laquelle

A a la signification donnée précédemment, le dérivé de 4-thia-β-$PGI_1$ obtenu, répondant à la formule générale I, pouvant être transformé par oxydation en un composé 4-sulfinyl-$PGI_1$ correspondant de formule générale I, et/ou par estérification, salification, amidation et hydrolyse et/ou réduction en un autre composé de formule générale I.

8. Procédé selon la revendication 7, caractérisé en ce que l'oxydation du dérivé 4-thia-β-$PGI_1$ de formule générale I en un dérivé 4-sulfinyl-$PGI_1$ correspondant est effectuée à l'aide d'acide 3-chloroperbenzoïque.

9. Préparations pharmaceutiques qui contiennent à titre de substance active un ou plusieurs composés selon l'une des revendications 1 à 6.

**Revendications** (pour l'Etat contractant AT)

1. Procédé pour la préparation de dérivés 4-thia- et 4-sulfinyl-$PGI_1$ de formule générale I

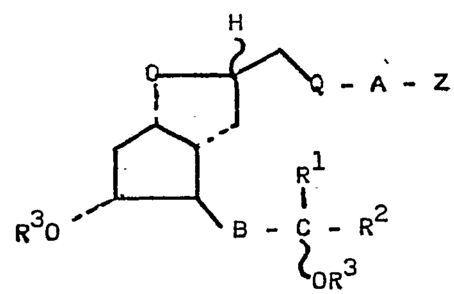

(I)

dans laquelle

Q représente —S— ou —SO—,

A représente un groupe alkylène en $C_1$ à $C_6$ linéaire ou ramifié,

B représente un groupe éthylène, vinylène ou éthynylène,

$R^1$ représente de l'hydrogène ou un radical alkyl en $C_1$ à $C_4$,

$R^2$ représente un radical alkyl en $C_1$ à $C_6$ linéaire ou ramifié, ou un radical aryloxyméthyl éventuellement monosubstitué par un atome de chlore ou un groupe méthoxy,

$R^3$ représente de l'hydrogène ou un radical acétyl, et

Z représente un radical —COOH, —CN, —$CH_2$OH ou —COOW où W représente l'équivalent d'un cation pharmaceutiquement acceptable ou un radical alkyl en $C_1$ à $C_4$, caractérisé en ce qu'un composé halogéné de formule générale II

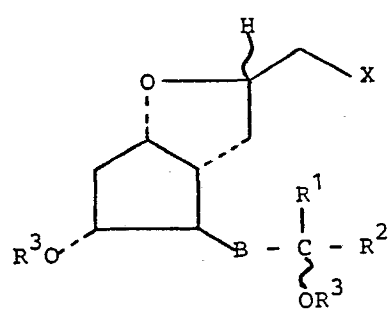

(II)

dans laquelle

$R^1$, $R^2$, $R^3$ et B ont les significations données précédemment, et

X représente un atome de brome ou d'iode,

a) est mis en réaction avec une thiourée, un sulfure de métal alcalin, un hydrogénosulfure de métal alcalin, de l'hydrogène sulfuré ou un sel d'acide mercaptoacétique, en ce que l'on acidifie et en ce que le thiol obtenu, répondant à la formule générale III

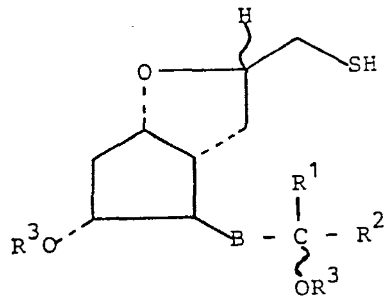

(III)

dans laquelle

$R^1$, $R^2$, $R^3$ et B ont les significations données précédemment, après addition de base, est mis en réaction avec un dérivé éthylénique de formule générale V

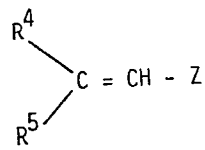

(V)

dans laquelle

Z a la signification donnée précédemment, et

$R^4$ et $R^5$ représentent indépendamment l'un de l'autre de l'hydrogène ou un groupe alkyle en $C_1$ à $C_3$, ou avec un halogénure d'alkyl de formule générale VI

$$X—A—Z \quad (VI)$$

dans laquelle

A, Z et X ont les significations données précédemment, ou bien

b) pour obtenir un dérivé de formule générale I, dans laquelle Z représente le radical —COOH, est mis en réaction avec un sel d'un acide mercaptocarboxylique de formule générale VII

$$HS—A—COOH \quad (VII)$$

dans laquelle

A a la signification donnée précédemment, le dérivé de 4-thia-β-$PGI_1$ obtenu, répondant à la formule générale I, pouvant être transformé par oxydation en un composé 4-sulfinyl-$PGI_1$ correspondant de formule générale I, et/ou par estérification, salification, amidation et hydrolyse et/ou réduction en un autre composé de formule générale I.

2. Procédé selon la revendication 1, caractérisé en ce que l'oxydation du dérivé 4-thia-β-$PGI_1$ de formule générale I en un dérivé 4-sulfinyl-$PGI_1$ correspondant est effectuée à l'aide d'acide 3-chloroperbenzoïque.

3. L'usage d'un ou de plusieurs des composés selon la revendication 1 ou 2 pour la production de préparations pharmaceutiques.